# EUROPEAN PATENT APPLICATION

(11) **EP 1 961 385 A1**
(43) Date of publication of application: **27.08.2008**
(21) Application number: 07290224.0
(22) Date of filing: 21.02.2007
(51) Int. Cl.: A61B 8/08

(54) **Combined nuclear and sonographic imaging apparatus and method**

(71) Applicant: European Organisation for Nuclear Research CERN, 1211 Geneva 23 (CH); SuperSonic Imagine, 13857 Aix-en-Provence Cedex (FR); Université de la Méditerranée, 13284 Marseille Cédex 07 (FR); Assistance Publique Hopitaux de Marseille, 13354 Marseille Cedex 5 (FR)
(72) Inventor: Cohen-Bacrie, Claude, 13122 Ventabren (FR); Mundler, Olivier, 13008 Marseille (FR); Lecoq, Paul, 01170 Chevry (FR)
(74) Representative: Liesegang, Eva

(57) **Abstract**

The present invention relates to an apparatus for use in medical imaging and a medical imaging method. The apparatus comprises nuclear medicine imaging means including detector means for detecting radiation as emitted upon decay of a radiopharmaceutical injected into a living object's body as well as means for generating a nuclear medicine image of said living object based on the detected radiation. The apparatus further comprises ultrasonography imaging means for also generating an ultrasonography image of the same living object.

## Description

The present invention relates to an apparatus for use in medical imaging and to a medical imaging method. In particular, the invention relates to an apparatus comprising nuclear medicine imaging means comprising detector means for detecting radiation as emitted upon decay of a radiopharmaceutical injected into a living object's body and means for generating a nuclear medicine image of said living object based on the detected radiation.

Well-known examples of such nuclear medicine imaging means are positron emission tomography (PET) devices or a single photon emission computed tomography (SPECT) devices. While the invention applies to all of the above mentioned nuclear medicine imaging techniques, for illustrative purposes the invention shall be explained with specific reference to PET.

PET is a nuclear medicine tomographic imaging technique using γ-rays. To conduct a scan, a short-lived radioactive tracer isotope which decays by emitting a positron and which has been chemically incorporated into a metabolically active molecule is injected into a living object such as an animal or a human patient, typically into the blood circulation. There is a waiting period while the metabolically active molecule becomes concentrated in the tissues of interest. Thereafter, typically the whole body of the patient is placed in a full-body imaging scanner. But dedicated apparatus have also been developed for the examination of specific organs (breast, prostate, heart, etc.)

As the radioisotope undergoes positive beta-decay, it emits a positron which after a short travel encounters and annihilates with an electron, thereby producing a pair of γ-photons, each having an energy of 511 keV and usually traveling in opposite directions. The γ-photons are detected by a radiation detector which is typically ring-shaped and surrounds the whole body of the patient. PET depends on simultaneous or coincident detection of the pair of γ-photons. Photons which do not arrive in pairs (i.e. within a few nanoseconds of one another) are ignored.

Since most of the γ-photons are emitted at 180° from one another during electron-positron annihilation, the source of radiation can be located along a straight line connecting the two radiation detector sites at which coinciding hits are detected. If the response of the radiation detector and the corresponding read-out electronics is fast enough, it is moreover possible to calculate the location of the radiation source on said line from a difference in arrival times. This method is called "time-of-flight" (TOF) measurement. However, this requires a time resolution of the measurement in the picosecond range which is currently difficult to achieve. Instead, statistics may be collected from tens-of-thousands of coincidence events and equations for the total activity of each parcel of the tissue of interest along the above mentioned straight line is solved, such that a map of radioactivity can be constructed and outputted.

An apparatus according to the preamble of claim 1 is known from P. Lecoq and J. Varela, "Clear-PEM, a dedicated PET camera for mammography", Nucl. Instr. & Methods in Phys. Research, Sec. A, vol. 486, pp 1- 6 (2002).

While nuclear imaging like PET is the only imaging modality providing clinical and preclinical imaging on a molecular level, it suffers from a poor anatomic resolution and does not provide structural information. In order to overcome this drawback, recently so-called multi-modality machines have been developed, in which nuclear imaging functionality and X-ray computed tomography (CT) functionality is combined. In such a multi-modality machine, PET in combination with a CT scanner can provide fused imaging showing in the same reference coordinate system a morphological/anatomical and a functional view of the domains of interest.

However, conventional whole-body PET scanners only have a resolution of a few millimeter up to a centimeter and are therefore not suitable for detecting cancer in the very early stage, where the cancer may be only for example 2 mm in diameter. Finally, exposing a patient to X-ray radiation is harmful to the patient as well.

It is an object of the present invention to provide a medical imaging apparatus and method which overcome the above-mentioned drawbacks.

This object is achieved by an apparatus according to claim 1 and by a method according to claim, 35. Preferable embodiments are defined in the dependent claims.

According to the invention, the apparatus further comprises ultrasonography imaging means for also generating an ultrasonography image of the same living object, It is to be understood that the term "image" and "imaging" is not limited to generating a graphical representation of the living object. The graphical representation is only one aspect of the image. In the context of the present invention, the term "imaging" more generally refers to associating one or more types of parameters or physical quantities with a corresponding point or region in space.

By combining nuclear medicine imaging with ultrasonography imaging, both, functional and anatomical information can be obtained simultaneously. As compared with a combined PET-CT apparatus, this can be achieved at much lower costs and without exposing a patient to ionizing X-ray radiation. Moreover, as will be explained in detail below, combining nuclear and ultrasonic imaging techniques in a single apparatus does not only provide the generic advantages of each technique by itself Instead, there is a number of synergetic effects which allows for a significant reduction of ionizing radiation exposure to the patient, enhanced image resolution and, for certain applications, unprecedented diagnostical reliability.

In one embodiment, the ultrasonography image may represent the echogeneicity, i.e. ultrasound reflectivity, and/or the velocity of moving tissue or fluids of said living subject.. This corresponds to ordinary ultrasonography and Doppler sonography, respectively. Additionally or alternatively, in a preferred embodiment the ultrasonography image represents viscoelastic properties of the tissue of said living object. The viscoelastic properties may comprise one or more of the following parameters of the tissue: shear modulus, Young's modulus, dynamic shear viscosity and mechanical impedance of the tissue. Displacements of tissue, differential displacements, non-linear reflectivity and other elasticity properties may also be represented on the ultrasonography image.

Ultrasonography imaging of viscoelastic tissue properties is a very recent technique which is for example described in US 5,606,971, US 2005/0252295 A1 and US5,810,731. This technique is based on the observation that during pathological and physiological processes in a living body, the viscoelastic properties of the tissue can dramatically change. Accordingly, by generating a viscoelasticity image, lesions or cancers can be diagnosed.

Preferably, the ultrasonography imaging means comprise means for generating a shear wave in the tissue of the living object, such as a transducer adapted to generate a focused ultrasound wave. In addition, the ultrasonography imaging means preferably comprises means for determining at least one propagation parameter of the shear waves, and means for determining the viscoelastic properties using said at least one propagation parameter. Herein, the term "determining" can refer to an exact calculation or a qualitative estimation. The at least one propagation parameter may comprise one or more of the following parameters: shear wave velocity, shear wave attenuation coefficient, amplitude and velocity of shear displacement of the tissue and/or the spatial and temporal dependencies thereof.

Preferably, the apparatus further comprises a control unit for associating the nuclear medicine image and the ultrasonography image with a common reference coordinate system. This allows to have simultaneously functional and morphological/anatomical information for each region of the living object under examination.

Preferably, the apparatus further comprises electronic data processing means adapted to determine a correlation between one or more aspects of the nuclear medicine image with one or more aspects of the ultrasonography image. For example, the correlation between a PET image and a shear modulus image can be examined for correlation using some image correlation algorithm as known per se from signal and imaging processing. Bright spots in the PET image correspond to a high concentration of radioisotopes. If, for example, ¹⁸F-fluoro-deoxy-glucose (FDG) is used as a radiopharmaceutical, a high concentration of FDG can be an indication of malignant tissues due to the much higher glucose consumption of cancerous cells as compared to normal tissue. However, there are also other causes for high FDG uptake, such as inflammatory processes as well as high muscular activity. However, a cancerous zone will also have a highly increased shear modulus as compared with a healthy zone revealing stiffness. If in fact a cancerous zone is present, this can also be reflected in the shear modulus image. Accordingly, a high correlation in the radioactivity and shear modulus images at a given spot could be interpreted as an indication of cancer with a specificity that is way beyond the specificity achievable with each of the techniques alone. This is one out of many other examples of the aforementioned synergy of the apparatus achieved by the invention, leading to a significant increase of the specificity of the combined examinations Further domains, for which an increased specificity will be beneficial, are cardiology and prostate examination.

Note that the above expression "one or more aspects of" indicates that, in the imaging scheme of the invention, a number of parameters or values may be associated with each point in space, some of which are obtained by nuclear imaging (such as the concentration of radioisotope) and some of which are obtained by ultrasonography imaging (such as tissue density, echogeneicity, velocity of moving tissue and one or more of the above-mentioned viscoelastic properties). By the combined nuclear and sonographic imaging that may give bi-or tri-dimensional information, a high dimensional image can be obtained. Each subset of the high dimensional parameter space can be examined for mutual correlations which allows for novel diagnostic techniques and criteria.

Preferably, the apparatus further comprises display means for simultaneously displaying a graphical representation of one or more aspects of a certain section of the nuclear image and a graphical representation of one or more aspects of the corresponding section of the ultrasonography image. In a preferred embodiment, the display means are adapted for displaying said two graphical representations next to one another or in a superposed fashion. Then, by simply looking at the simultaneously displayed graphical representations correlations and/or abnormalities can be recognized by an experienced physician with the naked eye.

Traditionally, radiopharmaceuticals are injected to a patient's or animal's blood circulation and are thus distributed through the whole body. This is disadvantageous for the following reasons. After injection, the radiopharmaceutical will generally accumulate at all metabolically active regions of the patient. If, however, only some smaller part of the patient' body is to be examined, such as a female breast or a thyroid, the overall dose injected to the patient is much higher than necessary for the local examination, since most of the radiopharmaceutical will also be located at places which are not under examination. Moreover, the radiation emitted by the FDG at those remote locations which are not under examination will impose noise on the image taken at the specific site of examination, which will lower the quality of the image.

In order to circumvent this problem, in a preferred embodiment, the ultrasonography imaging means are adapted to guiding microbubbles or nanoparticles containing radiopharmaceuticals by interaction with ultrasonic waves to a target position within the living object. For example, the microbubble or nanoparticle can be pushed by the ultrasound wave such as to move to a desired location. Preferably, the ultrasonic imaging means are further adapted to generate a suitable ultrasonic wave for breaking said microbubble or nanoparticle such as to free the radiopharmaceuticals contained therein. This allows to apply the radiopharmaceutical only where it is needed and to dramatically decrease the overall dose of radioactivity. Also, this helps to suppress noise in the nuclear image. Note that this technique can also be used to guide ultrasound contrast agents to the region of interest and is a further example of synergy obtained by combining nuclear and ultrasound imaging in a single apparatus.

Nuclear medicine is potentially the most promising imaging modality for molecular diagnostic. Combining nuclear medicine with ultrasonography imaging allows the use of specific targeted contrast agents that can accumulate in a certain region in the body. These agents are typically microbubbles containing therapeutic agents. The application of ultrasound using ultrasound emitting means bursts these bubbles which release the drug at a desired location. PET or other nuclear medicine imaging modality can be used to monitor the effect of the treatment or can be used to locate the site where the drug should be released.

Further ultrasound emitting means can be used to emit high energy ultrasound focusing schemes to burn or increase significantly the temperature of the tissue. High Intensity Focused Ultrasound (HIFU) may thus be implemented while monitoring target contrast agent delivering drugs using nuclear imaging means.

PET can thus be used for monitoring of the blood flow and FDG radiations in the lesion while doing therapy with HIFU. Moreover, combination of PET imaging and ultrasonographic imaging enables to use PET to plan an HIFU treatment. The PET modality can also be used as a monitoring and control tool to check the effect of the HIFU treatment.

In one embodiment, the detector means of the nuclear medicine imaging means comprises an ensemble of scintillator crystals sensitive to radiation emitted upon decay of the radiopharmaceutical. The detector means may further comprise photodetector means arranged to receive light emitted from the scintillator crystals. Preferred examples of said scintillator crystal comprise individual pixels or monolithic blocks of lutetium or mixed lutetium yttrium oxi-orthosilicate (LSO, LYSO), lutetium or mixed lutetium yttrium aluminum perovskite (LuAP, LuYAP), bismuth germanate (BGO), gadolinum or mixed lutetium gadolinum othosilicate (GSO, LGSO) and lanthanum bromides (LaCl₃, LaBr₃). The photodetector may be either a single or a multi-channel photomultiplier tube (PMT), a hybrid photomultiplier (HPMT), an avalanche photodiode (APD), a single photon avalanche photodiode (SPAD) or a Geiger mode avalanche photomultiplier tube (SiPMT). Alternatively, the detecting means may comprise solid state detector elements capable of collecting electric earners directly produced by the interaction of said radiation with said detector elements.

According to one embodiment, the nuclear imaging means further comprise read-out circuitry for receiving electronic detection signals from said photodetector elements or from said solid state detector elements and for generating from each detection signal one or more signals encoding the time stamp and the energy of the radiation hitting the scintillator crystal or solid state detector element, respectively. Encoding the time stamp is for example important for coincidence detection which is needed in PET applications. By encoding the energy of the detected radiation, it is possible to filter out background from scattered events by their energy as this energy differs from the energy expected from unscattered events.

In a preferred embodiment, the nuclear imaging means further comprise means for correcting the nuclear imaging data for the attenuation of said radiation based on a tissue density map as provided by the imaging ultrasonic parameters determined thanks to the ultrasonography imaging means. This is a further example of the synergy between the two imaging schemes combined in the apparatus of the invention. Namely, the tissue density will affect the intensity of γ-radiation detected at the detector and will accordingly skew the nuclear image. However, when used in combination with the ultrasonography device, the tissue density can be determined by ultrasonography and the expected attenuation of the γ-rays can be calculated or at least estimated. Then, the nuclear image can be corrected by accounting for the calculated attenuation.

In a preferred embodiment, the apparatus is specifically adapted for imaging a certain part of a human patient's body. Usually combined PET-CT scanners are full-body scanning devices which are expensive and bulky and not adapted to systematic screening. Also, because of their open geometry, they are very sensitive to background radiation. For example, if a female breast is to be examined in a full-body PET scanner, the image is severely affected by the background from the chest which reduces the sensitivity in the breast. Compared with a whole-body PET system, dedicated equipment specifically adapted to a certain human body part allows for better spatial resolution, obtained with fine-grain crystal segmentation, and allows tighter coverage of the region under analysis leading to a better sensitivity. Of course, sensitivity plays a pivotal role in PET imaging, since a high sensitivity allows a lower injected dose and a shorter examination time.

Preferably, the apparatus comprises means for immobilizing said certain body part. In a preferred embodiment, said certain body part is a female breast. As will be explained in more detail below with reference to a specific embodiment, mammography is one field where the apparatus of the invention will be highly useful, both with regard to reliability of the diagnosis and moderate equipment costs.

Preferably, the immobilizing means are capable of compressing the breast. In one embodiment, the immobilizing means may comprise two plates between which the breast is held. The two plates are preferably arranged vertically and substantially parallel to one another. At least one of the plates may be formed by a stretched foil, such as a mylar foil. The ultrasonography imaging means may comprise a transducer which is moveable along one of the plates, allowing for a projection image.

In an alternative embodiment, the immobilizing means may comprise a cone or a cylinder for accommodating the breast. The cylinder or cone may be made from kevlar. Preferably, the ultrasonography imaging means comprise a transducer that is rotatable around the surface of the cone or cylinder. This will allow for obtaining a tomographic ultrasound image. Moreover, this structure will allow a detailed radial imaging of the region around the nipple following the anatomy of the ducts which are the most critical portions in mammography. Preferably, the detector means comprise a ring-like assembly of detector elements at least partly surrounding the breast when immobilized by said immobilization means. Alternatively, the detector means may comprise two detector plates which are rotatable around the breast when immobilized by said immobilization means.

While the female breast is a very important example of a body part for which a specifically dedicated apparatus is provided, there are other body parts for which this is equally true, such as a thyroid or prostate. Also, the apparatus of the invention is applicable to further indications not related to cancer such as cardiac, vascular, abdominal (kidneys"pancreas, liver, colon), genital (prostate, ovarian, uterus, etc...) imaging. Another field of application is small animal imaging. This concerns in part, while not exclusively, all molecular imaging studies for the animal modeling of disease for drug development.

### DESCRIPTION OF THE PREFERRED EMBODIMENT

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates.

### SHORT DESCRIPTION OF THE FIGURES

- Fig. 1: is a conceptual drawing of an apparatus according to a preferred embodiment of the invention specifically adapted to mammography;
- Fig. 2A: is an enlarged view of two immobilization plates and a female breast held there between as shown in fig. 1;
- Fig. 2B: is a further enlarged view of the two immobilization plates and the female breast as seen from the right in fig. 1;
- Fig. 3: is a sectional side elevational view showing an immobilization cone accommodating a female breast and an ultrasound probe that is rotatable around the surface wall of the immobilization cone.

In the following, a specific embodiment of the apparatus of the invention will be described with reference to the accompanying drawings. The embodiment described below is an apparatus specifically adapted for mammography. While the invention is by no means limited to this specific device, it is nevertheless an important application of the invention and is particularly suitable for highlighting many of the advantages and innovative features of the invention.

As is well known, early detection of breast cancer is becoming a priority in health care policy of an increasing number of countries. A very large number of women (about 1 woman in 8) will develop breast cancer, which is the second leading cause of cancer death. However, after introduction of breast cancer screening by mammography on a regular basis, according to the analysis of clinical data, a reduction of roughly 30 % in the mortality by breast cancer could be achieved.

In ordinary mammography, low-dose X-rays are used to examine the human breast to look for different types of tumors and cysts. Unfortunately, the specificity of conventional X-ray mammography is rather low. The reason is that breast cancer is very often characterized by low contrast structures in the domain of low-energy X-rays, which leads to a low sensitivity of the order of 30 %. This is particularly true for the 40 % of women having dense breasts, for which X-ray mammography misses about 50 % of cancers. Dense breasts are seen in young patients and in older patients under hormone replacement therapy (HRT).

The high rate of false positive diagnosis leads to a large number of unnecessary biopsies. It has been observed that 60 to 85 % of the biopsies following an imaging indication obtained with X-rays or ultrasound do not correspond to malign pathology. The large number of unnecessary biopsies or even axillary dissections has a high cost for the society, not considering the psychological aspect on the women, Of course, even more dramatic is the too large number of false negative diagnosis with often fatal consequences for the patient. There is clearly a need for much higher sensitivity and specificity.

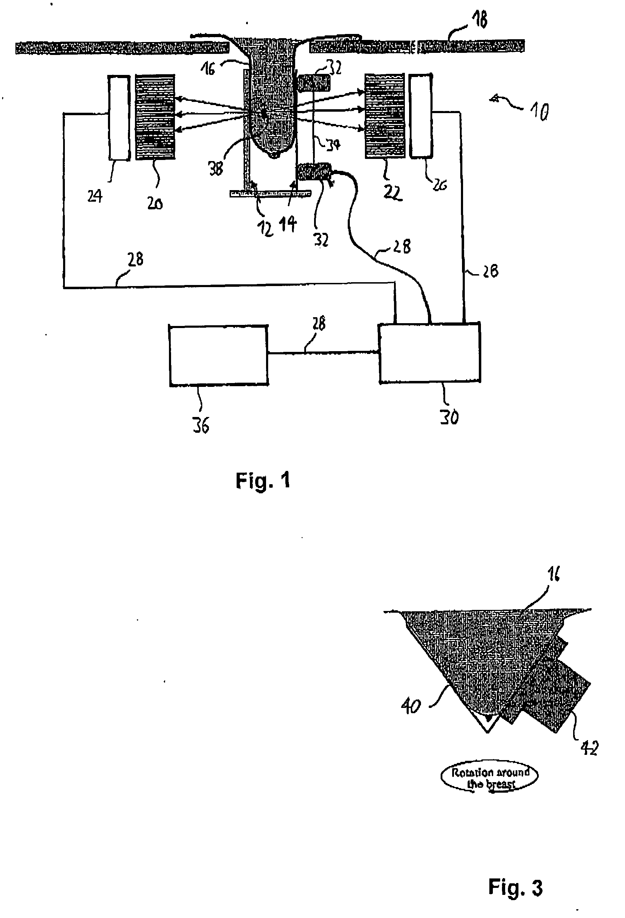

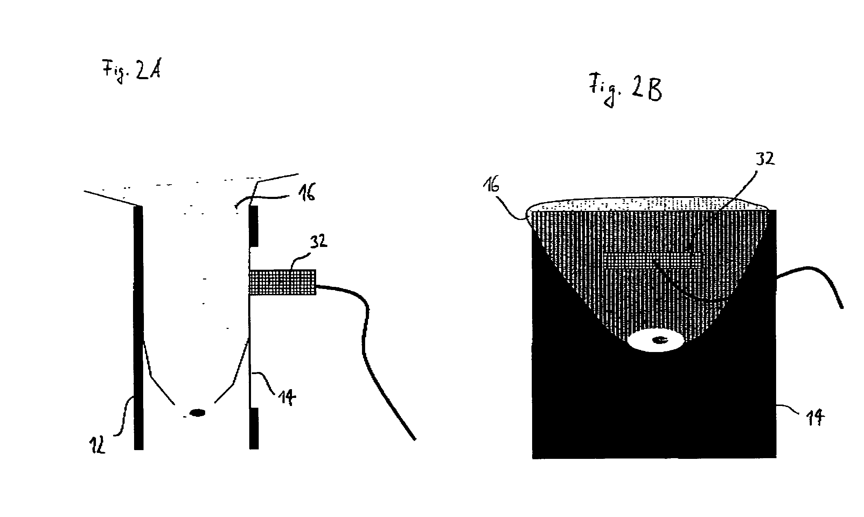

In fig. 1, an embodiment of the apparatus 10 of the invention is shown which is specifically adapted for mammography and which in particular allows for a much more reliable diagnosis of cancers. The apparatus 10 comprises immobilizing plates 12 and 14 between which a patient's breast 16 is held and pressed. The immobilizing plates 12 and 14 are arranged vertically and parallel to each other and are accommodating and compressing the breast 16 while a patient (not shown) lies on an examination table 18. In the embodiment shown in fig. 1, the immobilization plate 14 is a stretched mylar foil. An enlarged view of plates 12 and 14 with the breast 16 held therebetween and the ultrasound probe 32 is shown in fig. 2A. Fig. 2B shows a further view of the immobilized breast 16 as seen from the right in fig. 1.

The apparatus 10 comprises nuclear medicine imaging means, which in the specific example is a PET device. The PET device comprises two detector plates 20, 22, respectively, each one formed by a certain number of crystal matrices (for instance 8 x 4 crystals). In total, the number of crystals is about 3000 per plane covering an area of at least 10 x 12 cm². This allows for a resolution of the PET image of one or two millimeters. The detector plates 20, 22 are rotatable around the breast 16.

The detector plates 20, 22 also comprise corresponding photodetectors (e.g. avalanche photodiodes (APD) arrays (not shown)) for converting scintillation light generated in the crystal into an electrical detection signal. The photodetector array is connected with a corresponding read-out circuit 24, 26, respectively, which is connected to receive the electrical detection signals from the photodetector array. The read-out circuits 24, 26 are each connected via signal lines 28 with an electronic control unit 30, such as a computer.

In addition, the apparatus 10 comprises an ultrasound probe 32 which is moveable along plate 14 in a vertical direction by means of a probe holding and guiding means which is schematically represented by arrow 34. In fig. 1, the ultrasound probe 32 is actually displayed twice, namely at each of its extreme positions, respectively, During the move of the ultrasound probe 32, one may acquire 2D, 3D and even 4D information.

The ultrasound probe 32 comprises a transducer (not shown) for emitting ultrasonic waves through plate 14 into the breast 16, and receiver means (not shown) for detecting ultrasound waves received from breast 16 via plate 14. A coupling medium (not shown) is provided in order to enable the ultrasonic waves transmitted from the transducer to penetrate into the tissue.

The ultrasound probe 32 is advantageously also capable of generating a focused ultrasound wave for generating a shear wave in the tissue of breast 16. Ultrasound probe 32 is also connected with the electronic control unit 30 via a signal line 28. This signal line can also contain a wire that locates the position of the probe 32 on the holder 34. This enables the control unit 30 to identify in space the position of the probe for each transmit receive events. Finally, a display 36 is connected with the electronic control unit 30 via a further signal line 28.

Said control unit 30 centralizes images obtained from nuclear imaging means 20, 22, 24, 26 and from sonographic imaging means 32,

Nuclear imaging means and the stabilisation holder 12,14 are mechanically located one with respect to the other. The image obtained by the nuclear imaging means are located in the stabilization holder coordinate system. Furthermore, the holding and guiding means 34 of the probe 32 has precisely localized position with respect to the breast holder 12,14.

In the following, the operation of apparatus 10 will be explained. First of all, molecules involved in some metabolic processes and which are marked with positron-emitting isotopes, for example FDG, are injected into the patient. This can be done by injecting the marked molecules into the patient's blood circulation, such that they will spread over the whole body. Alternatively, the marked molecules are enclosed in a microbubble or nanoparticle and this microbubble or nanoparticle is directed by means of ultrasound waves generated by ultrasound probe 32 to a target location, where it is caused to explode by suitable ultrasonic excitation, such that the marked molecules are set free at the desired location.

In any case, the isotopes attached to the metabolically active molecules will emit a positron which after a short travel will encounter and annihilate with an electron. Upon the electron-positron annihilation, two γ-photons of 511 keV each will be emitted at 180° from each other. For illustration, three pairs of γ-photons are schematically shown by arrows in fig. 1, all of them originating from a small site 38 where there is a high concentration of metabolically active molecules. It is understood that a high concentration of metabolically active molecules can be an indication of a tumor.

The γ-photons emitted from site 38 each hit a crystal contained in the crystal arrays of detector plates 20 and 22, respectively, and give rise to a scintillation light flash. The scintillation light is received by a corresponding photodetector element of the photodetector array (not shown) and converted to an electrical detection signal which is read out by read-out circuitry 24, 26.

The read-out circuitry 24, 26 generates a signal encoding a time stamp and the energy of the detected event. The time stamp is used for detecting coincidence between two events, by which two γ-photons associated with the same electron-positron annihilation process are identified. If the energy of the event is within some expected energy window, it is passed by a filter (not shown) provided in the read-out circuitry 24, 26, otherwise it is blocked. This way, background noise is suppressed.

Since the apparatus 10 is specifically dedicated for mammography, its structure is optimized for it. In particular, the distance between the detector plates 20, 22 and the breast 16 is very small. The detector plates are adapted to tightly cover the region under analysis. Also, the fairly compact size allows a very hne-grain crystal segmentation. All of these features in combination allow for a image resolution of 2 mm or less. Also note that due to the structure specifically dedicated to mammography, the apparatus 10 avoids receiving radiation from decays occurring in the rest of the body, such that this source of noise is suppressed by the specific structure.

The two detector plates 20, 22 are rotated around the breast 16 to allow for detection in different directions, and the electronic control unit 30 generates a tomographic image from the detected events.

At the same time, sonographic images are taken using the ultrasound probe 32. Said ultrasound probe 32 emits ultrasonic waves and receives ultrasonic echoes from the tissue. Such echoes can be exploited in numerous ways potentially giving, for each point of the tissue, several parameters. These point of the tissue are identified as voxel in a volume located in the breast holder coordinate system. This holder 12,14 has a precise location with respect to the nuclear imaging means.

The sonographic image can thus be regarded as a "multi-dimensional" image, in that each point in space is associated with a number of parameters or physical values obtained during the ultrasonic scan. In the embodiment shown, using the ultrasound probe 32, for each point in space, the echogeneicity and the velocity of moving tissue and the shear modulus are detemined and displayed.

The electronic control unit 30 then fuses the PET data and the ultrasonic data to a common reference coordinate system of the breast holder. Accordingly, if for example the above mentioned region 38 is to be closely examined, for each point in said region there is information available from both types of imaging.

The combination of information from both types of imaging can be used in a number of ways, allowing for an improved reliability of diagnosis. For example, the same section of breast 16 can be displayed on display 36 simultaneously in a graphical representation of the radioactivity and of the echogeneicity. The echogeneicity image will provide morphological/anatomical information and the radioactivity image will provide the information of metabolic activity at the respective sites, which sites can be localized and identified from the echogeneicity image.

However, there are many more ways to compare or correlate data obtained from the two different imaging schemes, and this comparison and correlation is performed by the electronic control unit 30. For example, it would be possible to simultaneously display an image representing the radioactivity and an image representing the shear modulus in a given section of the breast. A region of high radioactivity could be an indication of a tumor, but this need not necessarily be the case. By also inspecting the shear modulus at the same place, the reliability of the prediction of a tumor can be enhanced. For example, if at the given site there is also a pronounced increase in the shear modulus, this will be a strong indication that there is a tumor.

By comparing and/or correlating different aspects of the nuclear image with different aspects of the ultrasonography image, it is possible to more reliably distinguish tumors from lesions and benign tumors from malign tumors.

Many modifications of the apparatus 10 of figs. 1 and 2 are possible. For example, instead of two rotating detector plates 20, 22, a ring-like detector could be used. Also, instead of using two parallel immobilization plates 12, 14, a conical receptacle 40 can be used, as shown in fig. 3. The conical receptacle 40 is made from Kevlar. In the embodiment of fig. 3, an ultrasound probe 42 is automatically rotatable around the axis of the conical receptacle 40, along the surface wall thereof. This arrangement will allow to obtain a high quality tomographic ultrasonography image of breast 16. Moreover, this structure is well-suited for visualizing the ducts which are located around the nipple of breast 16 and which are critical areas in mammography.

Although a preferred exemplary embodiment is shown and specified in detail in the drawings and the preceding specification, these should be viewed as purely exemplary and not as limiting the invention, It is noted in this regard that only the preferred exemplary embodiment is shown and specified, and all variations and modifications should be protected that presently or in the future lie within the scope of protection of the invention as defined in the claims.

### List of References

- 10: apparatus for use in medical imaging
- 12, 14: immobilizing plates
- 16: breast
- 18: examination table
- 20,22: detector plates
- 24,26: read-out circuitry
- 28: signal line
- 30: electronic unit control
- 32: ultrasound probe
- 34: movement range of ultrasound probe 32
- 36: display
- 38: examination site
- 40: immobilization cone
- 42: ultrasound probe

## Claims

1. Apparatus (10) for use in medical imaging, said apparatus (10) comprising nuclear medicine imaging means comprising detector means (20, 22) for detecting radiation as emitted upon decay of a radiopharmaceutical injected into a living object's body, and means (24, 26, 3 0) for generating a nuclear medicine image of said living object (16) based on the detected radiation,
**characterized in that** the apparatus (10) further comprises ultrasonography imaging means comprising ultrasound emitting and receiving means (32) spatially located regarding to the detector means, and means (34, 30) for also generating an ultrasonography image of the same living object (16) based on received ultrasounds.

2. Apparatus (10) according to claim 1, further comprising a control unit (30) for associating the nuclear medicine image and the ultrasonography image with a common reference coordinate system.

3. Apparatus (10) according to one of the preceding claims, further comprising electronic data processing means (30) adapted to determine a correlation between one or more aspects of the nuclear medicine image and one or more aspects of the ultrasonography image.

4. Apparatus (10) according to one of the preceding claims, comprising display means (30, 36) for simultaneously displaying a graphical representation of one or more aspects of a certain section of the nuclear medicine image and a graphical representation of one or more aspects of the corresponding section of the ultrasonography image.

5. Apparatus (10) according to claim 4, wherein said display means (30, 36) are adapted for displaying said two graphical representations next to one another or in a superposed fashion.

6. Apparatus (10) according to one of the preceding claims, wherein the nuclear imaging means further comprise means for correcting the nuclear medicine image data for the attenuation of said radiation based on a tissue density map provided by the ultrasonography imaging means.

7. Apparatus (10) according to one of preceding claims, wherein the ultrasonography image represents the echogeneicity and/or the velocity of moving tissue or fluids of said living object (16).

8. Apparatus (10) according to one of the preceding claims, wherein the ultrasonography image represents viscoelastic properties of the tissue of said living object (16).

9. Apparatus (10) according to claim 8, wherein said viscoelastic properties comprise one or more of the following parameters of the tissue: shear modulus, Young's modulus, dynamic shear viscosity and mechanical impedance of the tissue.

10. Apparatus (10) according to one of the preceding claims, wherein the ultrasound emitting and receiving means (32) comprise means for generating a shear wave in the tissue of said living object (16).

11. Apparatus (10) of claim 10, wherein said means for generating a shear wave in the tissue of said living object (16) comprise a transducer adapted to generate a focused ultrasound wave.

12. Apparatus (10) according to claims 10 or 11, wherein the ultrasonography imaging means comprise means for determining at least one propagation parameter of the shear wave, and means for determining said viscoelastic properties using said at least one propagation parameter.

13. Apparatus (10) according to claim 12, wherein said at least one propagation parameter comprises one or more of the following parameters: shear wave velocity, shear wave attenuation coefficient, amplitude and velocity of the shear displacement of the tissue and/or the spatial and temporal dependencies thereof.

14. Apparatus (10) according to one of the preceding claims, wherein the ultrasound emitting and receiving means (32) are adapted to guiding microbubbles or nanoparticles containing radiopharmaceuticals by interaction with ultrasonic waves to a target position (38) within said living object (16).

15. Apparatus (10) according to claim 14, wherein said ultrasound emitting and receiving means (32) are further adapted to generate a suitable ultrasound wave for breaking said microbubble or nanoparticle such as to free the radiopharmaceuticals contained therein.

16. Apparatus (10) according to one of the preceding claims, wherein the ultrasound emitting and receiving means (32) are adapted to generate high intensity focused ultrasound.

17. Apparatus (10) according to one of the preceding claims, wherein the nuclear medicine imaging means comprise a positron emission tomography (PET) device, a γ-camera or a single photon emission computed tomography (SPECT) device.

18. Apparatus (10) according to one of the preceding claims, wherein the detector means of the nuclear medicine imaging means comprise an ensemble (20, 22) of scintillator crystals sensitive to said radiation emitted upon decay of said radiopharmaceutical.

19. Apparatus (10) according to claim 18, wherein the detector means further comprise photodetector means arranged to receive light emitted from said scintillator crystals.

20. Apparatus (10) according to claim 18 or 19, wherein said scintillator crystals consist of individual pixels or monolithic blocks made from one or more of the following scintillating materials: lutetium or mixed lutetium yttrium oxyorthosilicate (LSO, LYSO), lutetium or mixed lutetium yttrium aluminum perovskite (LuAP, LuYAP), bismuth germanate (BGO), gadolinium or mixed lutetium gadolinium orthosilicate (GSO, LGSO), lanthanum bromides (LaCL₃, LaBr₃).

21. Apparatus (10) according to claim 19, wherein said photodetector means comprise single or multi-channel photomultiplier tubes (PMT), hybrid photomultipliers (HPMT), avalanche photodiodes (APD), single photon avalanche photodiodes (SPAD) or Geiger mode avalanche photomultiplier tubes (SiPMT).

22. Apparatus (10) according to one of the preceding claims, wherein the detector means comprise solid state detector elements capable of collecting electric carriers directly produced by the interaction of said radiation with said detector elements.

23. Apparatus (10) according to one of the preceding claims, wherein the nuclear imaging means further comprise read-out circuitry (24, 26) for receiving electronic detection signals from said photodetector elements (20, 22) or said solid state detector elements and for generating from each detection signal one or more signals encoding the time stamp and the energy of the radiation hitting the scintillator crystal (20, 22) or the solid state detector element, respectively.

24. Apparatus (10) according to one of the preceding claims, wherein the apparatus (10) is specifically adapted for imaging a certain part of a human patient's body.

25. Apparatus (10) according to claim 24, wherein said apparatus (10) comprises means (12, 14, 40) for immobilizing said certain body part.

26. Apparatus (10) according to claims 24 or 25, wherein said certain body part is a female breast (16).

27. Apparatus (10) according to claims 25 and 26, wherein said immobilizing means (12, 14, 40) are capable of compressing the breast (16).

28. Apparatus (10) according to claims 25, 26 and 27, wherein said immobilizing means comprise two plates (12, 14) between which the breast (16) is held.

29. Apparatus (10) according to claim 28, wherein the two plates (12, 14) are substantially vertical and/or substantially parallel to each other.

30. Apparatus (10) according to claim 28 or 29, wherein at least one of the plates (12, 14) is formed by a stretched foil (14), in particular a mylar foil.

31. Apparatus (10) according to one of claims 28 to 30, wherein the ultrasonography imaging means (32) comprise an ultrasound transducer which is moveable along one of the plates (14).

32. Apparatus (10) according to claims 25 and 26, wherein said immobilizing means comprise a cone (40) or cylinder for accommodating the breast (16).

33. Apparatus (10) according to claim 32, wherein said cone (40) or cylinder is made from Kevlar.

34. Apparatus (10) according to claims 32 or 33, wherein the ultrasonography imaging means comprise a transducer (42) rotatable around the surface of said cone (40) or cylinder.

35. Apparatus (10) according to claim 24, wherein certain body part is a thyroid or a prostate.

36. Method for medical imaging of a living object (16), said method comprising the steps of:
injecting a radiopharmaceutical into the living object's body;
detecting radiation emitted upon decay of said radiopharmaceutical;
generating a nuclear medicine image of said living object based on the detected radiation; and
additionally generating an ultrasonography image of the same living object (16).

37. Method of claim 36, further comprising a step of associating the nuclear medicine image and the ultrasonography image with a common reference coordinate system.

38. Method according to claims 36 or 37, further comprising a step of determining viscoelastic properties of the tissue of said living object (16) using ultrasound means (32).

39. Method according to one of claims 36 to 38, further comprising a step of determining a correlation between one or more aspects of the nuclear medicine image and one or more aspects of the ultrasonography image.
